# EUROPEAN PATENT SPECIFICATION

(11) **EP 1 523 678 B1**
(45) Date of publication and mention of the grant of the patent: **08.10.2008**
(21) Application number: 03765149.4
(22) Date of filing: 10.07.2003
(51) Int. Cl.: G01N 33/50, G01N 33/68, C12N 15/10

(54) **FUNCTIONAL SCREENING METHOD INVOLVING PROTEIN TRANSLOCATION/REDISTRIBUTION**
FUNKTIONELLES SCREENUNGSVERFAHREN MITTELS PROTEIN TRANSLOKATION/REDISTRIBUTION
PROCEDE DE CRIBLAGE FONCTIONNEL IMPLIQUANT LA TRANSLOCATION/REDISTRIBUTION DE PROTEINES

(30) Priority: 18.07.2002 GB 0216674
(43) Date of publication of application: 20.04.2005
(73) Proprietor: GE Healthcare UK Limited, Little Chalfont Buckinghamshire HP7 9NA (GB)
(72) Inventor: O'BEIRNE, Gerard Amersham Biosciences UK Limited, Cardiff CF14 7YT (GB); THOMAS, Nicholas Amersham Biosciences UK Limited, Cardiff CF14 7YT (GB)
(74) Representative: Franks, Barry Gerard
(86) International application number: PCT/GB2003/002983
(87) International publication number: WO 2004/009847

(56) References cited:
- WO-A-00/20859
- WO-A-00/68661
- WO-A-98/45704
- WO-A-99/35501

## Description

### Technical Field

The present invention relates to novel high-throughput functional genomic methods for determining gene and protein function in a cellular context. The method also has utility in identifying novel chemical modulators of gene and protein /enzyme activity.

### Background to the Invention

The large amounts of gene sequence, gene expression and protein expression data arising from the Human Genome Project, and from further downstream investigative efforts, have the potential to allow identification of many new drug targets. Realisation of this potential will require significant efforts in determining the function of new gene products and validating these proteins as drug targets.

Obtaining valid functional information on gene and protein function requires function to be determined (or confirmed) in-context; i.e. the function of the gene/protein should be determined in the presence of other genes/proteins which are likely to interact with it. Consequently there is a need for cell-based approaches for functional screening that enable functional information to be derived *in-situ* in a cellular environment where dynamic interactions between components may require other cellular components not available in a solution assay.

Moving high-throughput biology into cellular assays can build on and parallel previous work correlating and clustering transcription and interaction data derived from micro-array and protein-protein interaction studies (Ge *et al.* (2001) Nature Genetics *29*, 482-486). Aided by high throughput analysis technologies, cellular screening based approaches can begin to address the complexity necessary to unravel intracellular pathways and control in mammalian cells (Giese et al. (2002) Drug Discovery Today 7, 179-185), with the ultimate aim of producing sufficiently detailed description to allow a representation of cellular processes at a system level (Endy & Brent (2001) Nature 409, 391-395; Kitano (2002) Science 295, 1662-1664).

To achieve functional screening in a cellular context two elements are required;
a) genetic effector(s) or chemical modulator(s)
b) measurable phenotype(s); i.e. an assay read-out from a test system
to establish a cause-and-effect relationship between genes and phenotype or between chemicals and phenotypes. These elements can be used in a variety of screening processes that differ only in their objectives:
1) functional genomics; discovery of gene function in normal biology
2) target validation; discovery of gene function in aberrant biology
3) chemical genetics; discovery of chemicals that modulate normal phenotypes
4) drug discovery; discovery of chemicals that modulate aberrant phenotypes

In current procedures a test system is interrogated for the effects of genetic or chemical variance (i.e. up- or down-regulating expression of one gene, or the presence or absence of a candidate drug respectively), either alone or in combination. Consequently the effects (and by inference the function) of a gene (*effector*) or a drug (*modulator*) on a read-out from a test cell can be measured in isolation or in combination by observation of the behaviour of the test system. By using combinations of effectors and modulators of known and unknown function it is possible to begin to derive functional linkage between known and unknown entities and hence to assign function.

Use of cell-based assays for such screens (Croston (2002) Trends in Biotechnology 20, 110-5; Zheng & Chan 2002 Current Issues in Molecular Biology 4, 33-43) is becoming more widely adopted for reasons of acquiring contextual information as described above. Such assays employ a wide variety of assay methodologies, including reporter gene assays, cell growth, pre-cursor incorporation, cell transformation, cell morphology, and fluorescent enzyme assays. These approaches to functional screening have typically used pre-existing assays and instrumentation (e.g. measurement of a luciferase reporter gene in a luminometer) which require assay development prior to the screening process and which yield data averaged for a cell population under test.

US 6322973 (Iconix Pharmaceuticals) describes surrogate means for discovering chemical modulators of genes of unknown function. A heterologous gene of unknown function is expressed in a host cell (e.g. expression of a human gene in a yeast cell) and the host cell is evaluated for a resulting change in phenotype which can then be used as the basis of a cellular assay. Consequent exposure of the host cell exhibiting an altered phenotype to a test substance and assaying for an effect of the test substance on the cellular assay identifies test substances which are modulators of the function of the heterologous gene.

US 6340595 (Galapagos Genomics) describes means for identifying the function of the products of a library of sample nucleic acids by expression of the library of nucleic acids in adenoviral vectors. The sample nucleic acids are synthetic oligonucleotides, DNA, or cDNA.and encode polypeptides, antisense nucleic acids, or genetic suppressor elements. The sample nucleic acids are expressed in a host and the resultant altered phenotype used to assign a biological function to the product encoded by the sample nucleic acid. WO020274 (Rosetta Inpharmatics) describes methods and systems (e.g., computer systems and computer program products) for characterising cellular constituents, particularly genes and gene products. The invention provides methods for assigning or determining the biological function of uncharacterised genes and gene products by using response profiles derived from measurements of pluralities of cellular constituents in cells having a modified gene or gene product, as phenotypic markers for the gene product. Methods are provided for clustering such response profiles so that similar or correlated response profiles are organised into the same cluster. The invention also provides databases of response profiles to which the response profile of an uncharacterised gene or gene product are compared.

WO0171023 (Genetrace) describes methods for deciphering genetic function. The method provides a matrix of cell lines in which target-specific modified cell lines differ from parental cells in the activity or concentration of a selected protein or nucleic acid. The matrix of cells is exposed to one or more stimuli or test compounds and the cell matrix profiled for response(s) to the stimuli or test compounds. Analysis of the resulting profiles yields information on the genetic function of elements that differ in activity or concentration across the matrix of cells.

WO0068661 (Gordon) discloses methods of detection of cellular exposure to an addictive drug and methods for identifying a substance that can alter or mimic the cellular effects of an addictive drug (e.g. ethanol). One method involves determining the function or effect of a chemical modulator from a library of chemical modulators of known and unknown function on a population of cells comprising determining the distribution of an indicator nucleic acid, for example by measuring the cellular localization of an 'ethanol indicative protein' such as a Green Fluorescent Protein-tagged fusion protein, in the presence and the absence of a first and second chemical modulator.

All of the above prior-art methods are characterised by one or more of the following;
a) measurement of the effects of heterologous genes (e.g. human genes in yeast)
b) a requirement for development of suitable assays prior to screening
c) a requirement for engineered cell lines prior to screening.

A significant problem encountered in the prior art assays described above is that they rely on pre-existing assays and are thus, *a priori,* limited in scope, coverage of biological events being limited by the availability of known assays. This leads to the further problem that assignment of function is limited to those entities which interact with a biological process linked to an available assay read-out. Furthermore, since in general these assays report on cause and effect relationships averaged across a cell population, they do not yield information on the distribution of response across a cell population (e.g. due to cell cycle status, or due to a mixed population of responding and non-responding cells). An additional problem with the prior art methods is that the assays can only be used on stable populations of cells and are not generally suitable for use with nonhomogeneous populations of cells such as transiently transfected cells.

Consequently what is required to increase the efficiency of functional screening are methods which do not require pre-existing assays, have the broadest possible coverage of cellular processes and provide data at the individual cell level. The present invention provides methods for functional screening in which assays are generated in concert with screening in an iterative process which expands the scope of biological coverage with each iteration and which uses image-based analysis to yield data at sub-cellular resolution.

The method of the present invention circumvents at least some of the limitations of prior-art methods discussed above by providing means to generate functionally diagnostic assays which are integrated into a functional screening process. The method takes advantage of the fact that many cellular proteins exhibit a characteristic cellular localisation and in many cases change their cellular localisation in response to certain stimuli. Consequently, given collections of coding nucleic acid sequences and of chemical compounds, where both collections contain members of known and unknown function, it is possible to generate pairings of one nucleic acid sequence with one chemical compound to produce a specific cellular localisation of a marker coupled to the product of the nucleic acid sequence. Such pairings may then be used as diagnostic assays for testing against other collection members and thus build up clusters and linkages therebetween. In this way, using some members of each collection which are of known function, it is possible to assign function to previously uncharacterised elements by linkage to known elements. Thus the method of the present invention allows function to be assigned at a molecular and temporal level for any cellular component, chemical, drug or other active moiety which induces a change in behaviour of an endogenous or exogenous cellular component by reference to changes induced by other moieties of known function. Nondestructive single cell analytical methods are used to analyse the cellular behaviour of indicators influenced by genetic effectors and chemical modulators, where the indicators and effectors may be either endogenous or exogenous to the cell.

### Summary of the Invention

According to a first aspect of the present invention, there is provided a method for determining the function or effect of a genetic element or a chemical modulator from libraries of genetic elements and chemical modulators of known and unknown function on a population of cells, the method comprising
i) determining the distribution of a detectable label expressed from an indicator nucleic acid sequence in the cells in the presence and the absence of a first chemical modulator, which modulator affects the distribution of the indicator, wherein the cells are both co-expressing a library of effector nucleic acid sequences and are in the presence of a library of second chemical modulators; and
ii) analysing the distribution data from all combinations of the effector, modulator and indicator to derive functional linkages and assign function to the effector and the second modulator.

In the context of the present invention, the following terms are to be interpreted as defined below:
*'Effector' -* a nucleic acid sequence with biological function or activity, resulting either from an expressed protein with biological function or activity (e.g. cDNA or other coding nucleic acid sequence) or resulting from another mechanism of action (e.g. antisense and RNAi sequences);
*'Modulator' -* a chemical moiety with biological function or activity;
*'Indicator' -* a nucleic acid sequence which comprises a detectable label, encodes a detectable label or which may optionally be fused to a sequence encoding a detectable protein label and expressed in a cell resulting in a characteristic localisation of the detectable protein;
*'Cellular Assay'-* an assay providing a diagnostic read-out of the biological activity of an effector or modulator

In a second aspect of the present invention, there is provided a method for determining the function or effect of a genetic element or a chemical modulator from libraries of said genetic elements and chemical modulators of known and unknown function on a population of cells, the method comprising
i) determining the distribution of a detectable label expressed from an indicator nucleic acid sequence in said cells in the presence of a first chemical modulator, which modulator affects the distribution of the indicator, wherein the cells are both co-expressing a library of effector nucleic acid sequences and are in the presence of a library of second chemical modulators;
ii) comparing the distribution data of i) above with known distribution data, stored on an electronic or optical database, for the detectable label expressed from the indicator nucleic acid sequence in the absence of the first chemical modulator; and
iii) analysing the distribution data from all combinations of the effector, modulator and indicator to derive functional linkages and assign function to the effector and the second modulator.

Suitably, the effector nucleic acid sequence encodes a protein or peptide and is selected from the group consisting of DNA, cDNA, RNA and Protein Nucleic Acid. Preferably, the effector nucleic acid sequence is an antisense oligonucleotide (cf. Dean (2001) Current Opinion in Biotechnology, 12, 622-625). More preferably, the effector nucleic acid is a small interfering RNA (siRNA) which causes gene silencing (cf. Elbashir et al. (2002) Methods, 26, 199-213). RNA interference (RNAi) is a highly conserved gene silencing mechanism that uses double-stranded RNA as a signal to trigger the degradation of homologous mRNA. The mediators of sequence-specific mRNA degradation are 21- to 23-nt small siRNAs generated by ribonuclease III cleavage from longer double-stranded RNA. Preferably, there is provided an expression vector comprising suitable expression control sequences operably linked to an indicator or an effector nucleic acid sequence according to the present invention. The DNA construct of the invention may be inserted into a recombinant vector, which may be any vector that may conveniently be subjected to recombinant DNA procedures. The choice of vector will often depend on the host cell into which it is to be introduced. Thus, the vector may be an autonomously replicating vector, ie. a vector which exists as an extrachromosomal entity, the replication of which is independent of chromosomal replication, e.g. a plasmid. Alternatively, the vector may be one which, when introduced into a host cell, is integrated into the host cell genome and replicated together with the chromosome(s) into which it has been integrated.

The vector is preferably an expression vector in which the effector or indicator nucleic acid sequence is operably linked to additional segments required for transcription of the nucleic acid. In general, the expression vector is derived from plasmid or viral DNA, or may contain elements of both. Preferably, the expression vector is selected from the group consisting of plasmid, retrovirus and adenovirus. The term, "operably linked" indicates that the segments are arranged so that they function in concert for their intended purposes, e.g. transcription initiates in a promoter and proceeds through to protein synthesis.

The promoter may be any DNA sequence which shows transcriptional activity in a suitable host cell of choice, (eg. a mammalian cell, a yeast cell, or an insect cell) for transcription of the indicator or effector nucleic acid sequence. The promoter may be derived from genes encoding proteins either homologous or heterologous to the host cell.

Examples of suitable promoters for directing the transcription of the nucleic acid sequences of the invention in mammalian cells are the CMV promoter (US 5168062, US5385839), Ubiquitin C promoter (Wulff et al. (1990) FEBS Lett. 261, 101-105), SV40 promoter (Subramani et al. (1981) Mol. Cell Biol. 1, 854-864) and MT-1 (metallothionein gene) promoter (Palmiter et al. (1983) Science 222, 809-814). An example of a suitable promoter for use in insect cells is the polyhedrin promoter (US 4745051; Vasuvedan et al.(1992) FEBS Lett. 311, 7-11). Examples of suitable promoters for use in yeast host cells include promoters from yeast glycolytic genes (Hitzeman et al. (1980) J. Biol. Chem. 255, 12073-12080; Alber & Kawasaki (1982) J. Mol. Appl. Gen, 1, 419-434) or alcohol dehydrogenase genes (Young et al., in Genetic Engineering of Microorganisms for Chemicals (Hollaender et al, eds.), Plenum Press, New York, 1982), or the TP11 (US 4599311) or ADH2-4c (Russell et al. (1983) Nature 304, 652-654) promoters.

The effector and indicator nucleic acid sequences of the present invention may also, if necessary, be operably connected to a suitable terminator, such as the human growth hormone terminator, TPI1 or ADH3 terminators. The vector may further comprise elements such as polyadenylation signals (e.g. from SV40 or the adenovirus 5 Elb region), transcriptional enhancer sequences (e.g. the SV40 enhancer) and translational enhancer sequences (e.g. the ones encoding adenovirus VA RNAs).

The vector may further comprise a DNA sequence enabling internal ribosomal entry and expression of two proteins from one bicistronic transcript mRNA molecule. For example, the internal ribosomal entry sequence from the encephalomyocarditis virus (Rees S, et al. (1996) BioTechniques, 20, 102-110 and US 4937190).

The recombinant vector may further comprise a DNA sequence enabling the vector to replicate in the host cell in question. An example of such a sequence (when the host cell is a mammalian cell) is the SV40 origin of replication.

When the host cell is a yeast cell, examples of suitable sequences enabling the vector to replicate are the yeast plasmid 2µ replication genes REP 1-3 and origin of replication.

The vector may also comprise selectable markers, such as a gene that confers resistance to a drug, e.g. ampicillin, kanamycin, tetracyclin, chloramphenicol, puromycin, neomycin or hygromycin.

The procedures used to ligate the effector and indicator nucleic acid sequences of the invention, the promoter and optionally the terminator and/ or targeting sequence, respectively, and to insert them into suitable vectors containing the information necessary for replication, are well known to persons skilled in the art (e.g. Molecular Cloning, Sambrook & Russell, Cold Spring Harbour Press 2001).

Suitably, the indicator nucleic acid sequence comprises a detectable label or encodes a detectable label. Preferably, indicator nucleic acid sequence is created by fusing the nucleic acid sequence to a nucleic acid sequence encoding a detectable label.

Suitably, the detectable label is selected from the group consisting of fluorescent protein, enzyme, antigen and antibody.

Fluorescent proteins and fluorescent protein derivatives of chromoproteins have been isolated from a wide variety of organisms, including *Aequoria victoria, Anemonia* species such as *A. majano* and *A. sulcata, Renilla* species, *Ptilosarcus* species, *Discosoma* species, *Claularia* species, *Dendronephthyla* species, *Ricordia* species, *Scolymia* species, *Zoanthus* species, *Montastraea* species, *Heteractis* species, *Conylactis* species and *Goniopara* species.

The use of Green Fluorescent Protein (GFP) derived from *Aequorea victoria* has revolutionised research into many cellular and molecular-biological processes. However, as the fluorescence characteristics of wild type (native) GFP (wtGFP) are not ideally suited for use as a cellular reporter, significant effort has been expended to produce variant mutated forms of GFP with properties more suitable for use as an intracellular reporter (Heim et al., (1994), Procedings of the National Acadamy of.Sciences (USA), 91, 12501;. Ehrig et al., 1995, FEBS Letters, 367,163-6; WO96/27675; Crameri, A. et al., (1996), Nature Biotechnology 14, 315-9; US 6172188; Cormack, B.P. et al., (1996) Gene 173, 33-38; US 6194548; US 6077707 and GB Patent Number 2374868 ('Amersham Biosciences UK Ltd.'). Preferred embodiments disclosed in GB Patent No 2374868 comprise GFP derivatives selected from the group consisting of: F64L-V163A-E222G-GFP, F64L-S175G-E222G-GFP, F64L-S65T-S175G-GFP and F64L-S65T-V163A-GFP.

In a preferred embodiment, the fluorescent protein is a modified Green Fluorescent Protein (GFP) having one or more mutations selected from the group consisting of Y66H, Y66W, Y66F, S65T, S65A, V68L, Q69K, Q69M, S72A, T2031, E222G, V163A, 1167T, S175G, F99S, M153T, V163A, F64L, Y145F, N149K, T203Y, T203Y, T203H, S202F and L236R.

Preferably, the modified GFP has three mutations selected from the group consisting of F64L-V163A-E222G, F64L-S175G-E222G, F64L-S65T-S175G and F64L-S65T-V163 as disclosed in GB Patent Number 2374868.

Preferably, the enzyme is selected from the group consisting of β-galactosidase, nitroreductase, alkaline phosphatase and β-lactamase. The indicator nucleic acid sequence can thus be detected by the action of the enzyme on a suitable substrate added to the cell. Examples of such substrates include nitro-quenched CyDyes^{™} (Amersham Biosciences, nitroreductase substrate), ELF 97 (Molecular Probes, alkaline phosphate substrate) and CCF2 (Aurora Biosciences,β-lactamase substrate).

Suitably, the modulator is selected from the group consisting of organic compound, inorganic compound, peptide, polypeptide, protein, carbohydrate, lipid, nucleic acid, polynucleotide and protein nucleic acid. Preferably, the modulator is selected from a combinatorial library comprising similar organic compounds such as analogues or derivatives.

Suitably, the cell is a eukaryotic cell. Preferably, the eukaryotic cell is selected from the group consisting of mammal, plant, bird, fungus, fish, insect and nematode, which cell may or may not be genetically modified. More preferably, the mammalian cell is a human cell, which cell may or may not be genetically modified.

Preferably, the localisation of the detectable label is determined using an imaging system. A suitable Imaging System is the In Cell Analyzer, as described in WO 99/47963 and PCT/GB03/01816.

### Brief Description of the Invention

Figure 1; Schematic for generation of an indicator cell assay from a cDNA collection.
Figure 2; Schematic for establishing an inferred functional relationship between an effector and a modulator in a cellular assay.
Figure 3; Schematic for generation of an indicator assay from a cDNA collection and a chemical collection and subsequent application of selected indicator assays for establishing functional relationships between components of the two collections.
Figure 4: a) Triplet functional relationship between effector, modulator and indicator. b) variation in triplets derived from effector and modulator collections comprising components of known and unknown function and/or biological activity.
Figure 5; Schematic for establishing extended functional relationships between effector and/or modulators of known and unknown function through connection of triplet functional relationships through common components.
Figure 6; Image fluorescence intensity measurements for a nuclear DNA stain and EGFP-fusion protein expression for a range of cDNA indicators transfected into HeLa cells.
Figure 7; Image fluorescence intensity measurements for a nuclear DNA stain and EGFP- fusion protein expression from a single cDNA indicator transfected into HeLa cells.
Figure 8; Nuclear:cytoplasmic indicator distribution in HeLa cells exposed to dexamethasone and staurosporine.
Figure 9; Scatterplot of indicator distribution in HeLa cells exposed to dexamethasone and staurosporine.
Figure 10; Response of a range of indicators to staurosporine exposure of HeLa cells.
Figure 11; Effects of transient transfection of a range of cDNA effectors on distribution of a NFκB p65-GFP indicator in CHO cells.
Figure 12; Effects of transient transfection of a range of cDNA effectors on the response of a NFκB p65-GFP indicator to IL-1 stimulation in CHO cells.
Figure 13; Effects of transient transfection of a range of cDNA effectors on distribution of a Rac1-GFP indicator in CHO cells.

### Detailed Description of the Invention

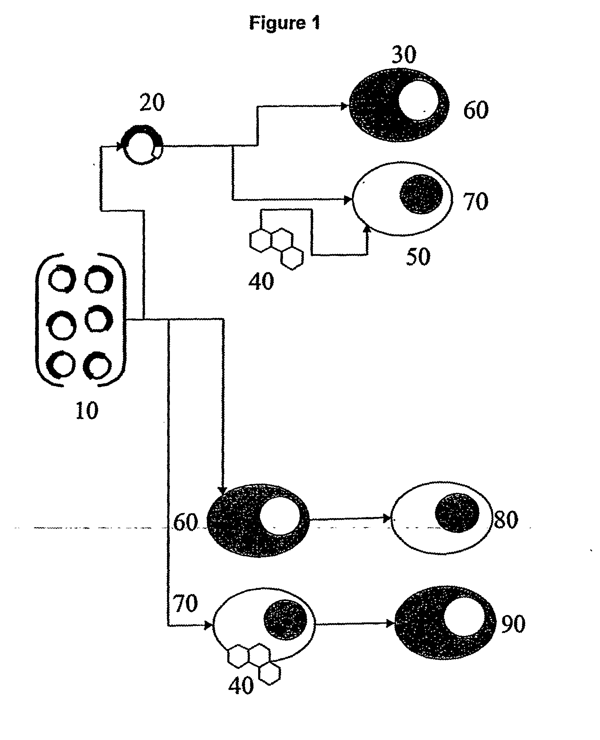

To achieve the method of the current invention one or more of a collection of nucleic acid sequences [10] (Figure 1) in a vector suitable for expression of the nucleic acid in a host cell are subcloned into a further vector [20] to provide fusions of the protein product of the nucleic acid sequence(s) with a detectable protein. The detectable protein may be any protein which may be expressed in a mammalian cell and detected using appropriate instrumentation. Suitable detectable proteins include fluorescent proteins such as Green Fluorescent Protein Expression of the fusion protein in mammalian cells may be achieved by use of standard methods including chemically mediated transfection (FuGENE, Roche; Lipofectin, Invitrogen), electroporation (Brunner et al. (2002) Molecular Therapy 5, 80-6) or ballistic delivery (Burkholder et al. (1993) J Immunol Methods 965,149-56).

Expression of the detectable fusion protein in a population of host cells [30] yields a distribution of the detectable protein characteristic of the distribution of the protein encoded by the nucleic acid sequence [10]. Expression of the fusion protein in a second population of host cells [50] in the presence of a test compound [40] will in certain circumstances yield a distribution of the fusion protein [70] which differs from that in the absence of the test compound [60]. In such cases of combinations of [20] and [40] which yield distribution patterns where [60] differs from [70] the particular combination of test compound and detectable fusion protein provide a basis for further investigations. It is an important aspect of this process that it does not require knowledge of the identity or biological function of either component [10] or [40] to be known, beyond that required to follow the process as described; e.g. sufficient sequence information for [10] to enable assembly of the fusion construct [20]. This process establishes combinations of fusion proteins [20] and test compounds [40] which together engineer a defined and responsive cellular phenotype, i.e. a cell-based assay which can be used in further functional screening.

Once key combinations of [20] and [40] have been established in which [40] exhibits a reproducible activity in modulating the cellular distribution of [20], a second round of screening may be carried out in which nucleic acid sequences [10] are transfected into cells expressing the detectable fusion protein in the absence [60] and presence [70] of the test compound [40]. Cells are subsequently evaluated for modulation of the engineered phenotype to identify nucleic acid sequences [10] which modulate the cellular distribution of the detectable fusion protein either alone [80], or in combination [90] (antagonism or synergy) with the test compound.

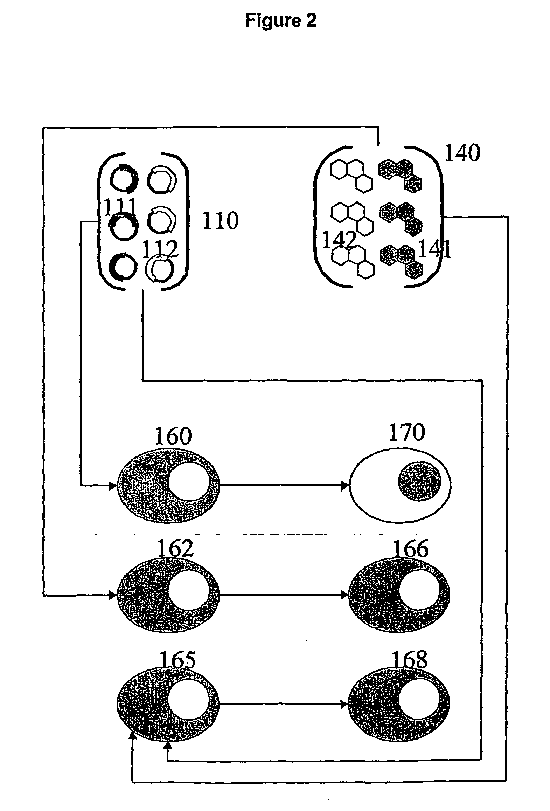

Repetition of the screening process (Figure 2) using libraries of nucleic acid sequences [110] and test compounds [140], where both libraries contain elements of known (shaded) [111] [141] and unknown (unshaded) function [112] [142], and exposing cells of engineered phenotype to elements of these libraries alone [160] [162] and in combination [165], allows the functions and interactions of nucleic acid sequences and test compounds to be investigated. In the example of Figure 2, interaction of a nucleic acid sequence component [170,166,168] of the library [110] with cells of engineered phenotype [160] causes a change in the detected phenotype [170]; interaction of a chemical component of the test compound collection [140] with cells of the same engineered phenotype [162] does not change the detected phenotype [166]; co-exposure of further cells of the same engineered phenotype [165] to the same chemical and genetic elements in combination does not lead to a change in the observed phenotype [168], indicating some form of antagonism between the functions of the test compound and the expressed nucleic acid sequence.

Large scale screening using a library of nucleic acid sequences of known and unknown function in combination with a collection of test compounds of characterised or uncharacterised biological activity can therefore be carried out to establish combinations of nucleic acid sequences and chemical entities which operate in concert to modify a detectable cellular phenotype measured by a cellular assay. Since the process inherently generates cellular assays the method has advantages over previously used approaches in that it does not require either prior knowledge of biological activities or pre-existing cell assays; although the process may be used in conjunction with pre-existing cell-based assays, where available.

A number of groups (Bejarano et al. (1999) J Cell Sci 112 (23), 4207-11; Misawa et al. (2000) Proc Natl Acad Sci U S A 97, 3062-6; Gonzalez et al. (2000) Trends Cell Biol 10, 162-5; Rolls et al .(1999) J. Cell Biol. 146, 29-44; Simpson et al. (2000) EMBO 1, 287-92) have reported using GFP tagging of unknown genes or sequence motifs arising from cDNA libraries or other sources to identify sequences associated with proteins of defined sub-cellular localisation. Developments are already in place to automate cloning (Rolls et al. (1999) J. Cell Biol. 146, 29-44) which allows high-throughput generation of the N- and C-terminal GFP fusions necessary for transfection.

Use of high throughput image based analysis using instruments such as the Amersham Biosciences IN Cell Analyzer (Goodyer et al. (2001), Society for Biomolecular Screening, 7th Annual Conference and Exhibition, Baltimore, USA *Screening and signalling events in live cells using novel GFP redistribution assays)* permits the use of assays measuring tagged protein localization to be carried out on transiently modulated cells, (e.g. by transient cDNA transfection) with data collected on an individual cell basis. This approach offers a number of benefits, including removal of the need to pre-establish stable indicator cell lines prior to screening yields assay results which are less likely to be distorted by 'over-expression squelching' and phenotype distortion arising through cellular selection (Giese et al Drug Discovery Today (2002) 7, 179-186) associated with generation of large numbers of stable cell lines.

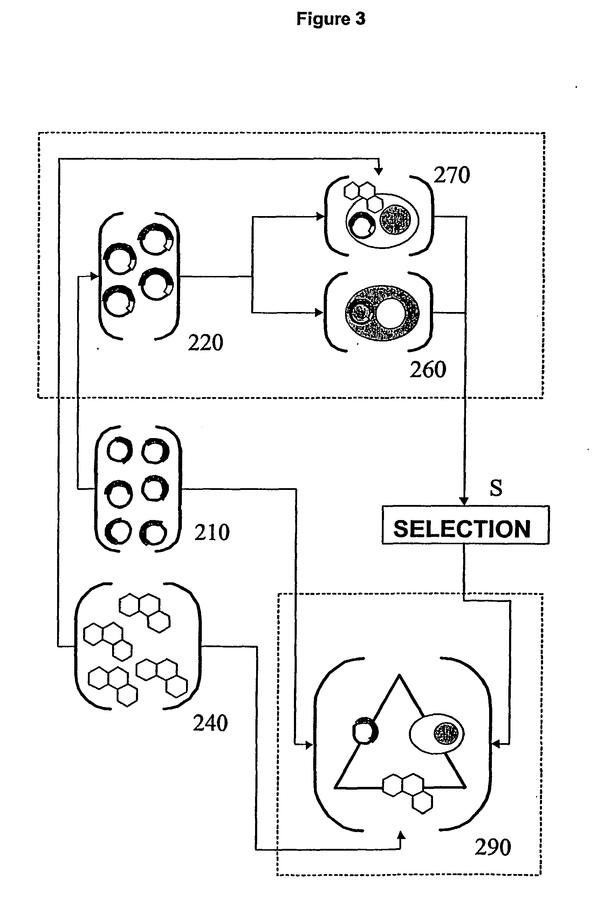

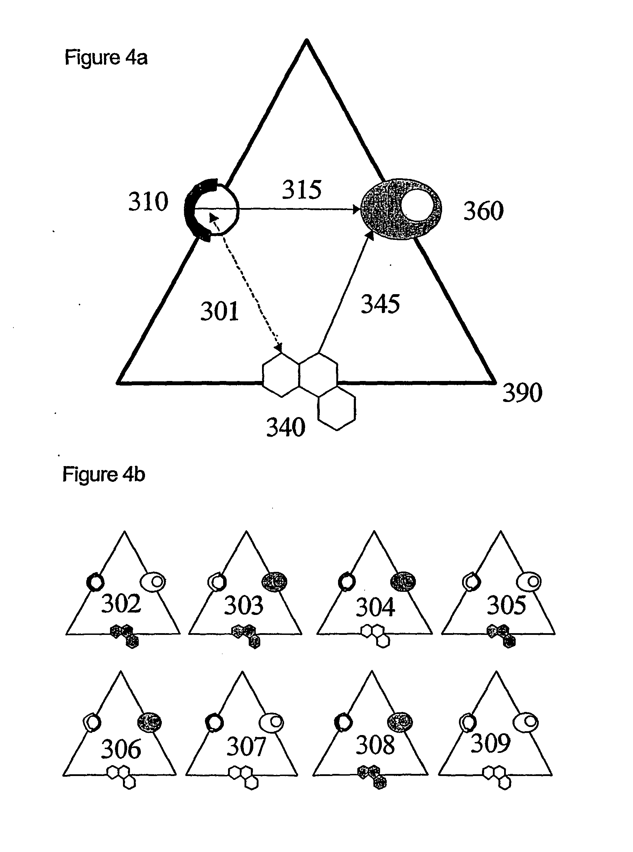

The method of the invention may be used to establish functional relationships between genetic elements (effectors), chemical elements (modulators) and cellular assays (indicators). Starting from collections of effectors [210] (Figure 3) and modulators [240] of known or unknown function, cDNA effectors are engineered as fusions with a detectable marker protein [220] and transfected into target cells in the presence [270] and absence [260] of selected modulators [240]. Combinations of effectors, modulators and target cells giving a reproducible difference in the localisation of the detectable fusion protein are selected [S] for further rounds of functional screening in which the selected combinations are challenged with effectors [210] or modulators [240]. By this means many three-way combinations of effectors, modulators and indicators may be tested [290]. Tri-partite combinations [390] (Figure 4a) in which the activity [345] of a chemical modulator [340] and the activity [315] of a genetic effector [310] on a indicator cell based assay [360] are correlated and used to infer the presence or absence of a functional linkage [301] between effector and modulator, may be used to establish functional links and clusters between many different entities. For any collections of effectors and modulators where the biological function or activity of components of the collections are both known and unknown, and where these collections are tested in combination with indicator cell assays of a known (i.e. pre-existing assays) or unknown biological significance, eight possible three-way combinations (triplets) are possible [302]-[309], and are summarised in Table 1.

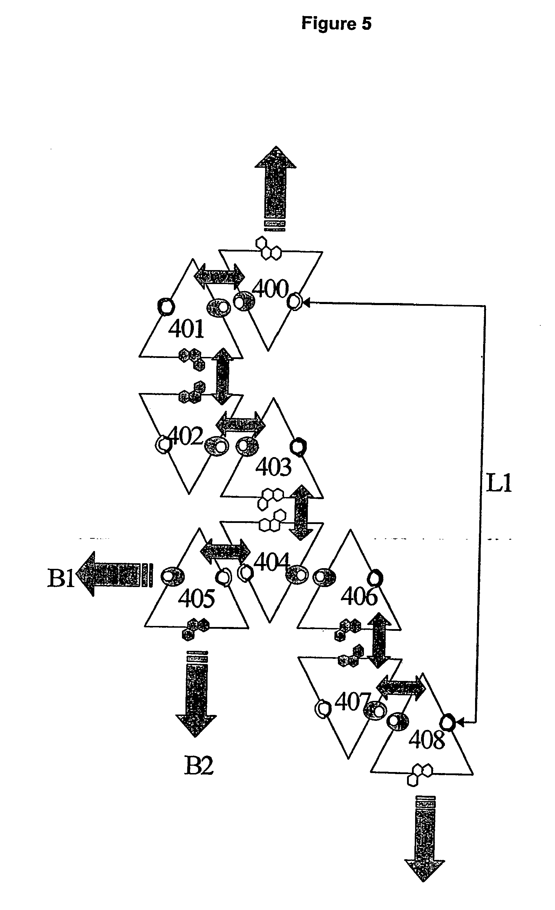

Consequently by collecting data from a large number of triplets where unknown elements are tested in combination with known elements and selecting triplets in which there is an interaction between all three components it is possible to assemble networks of functional linkages which yield information on the biological function of previously uncharacterised elements. For example a triplet [400] (Figure 5), in which the biological activities of both effector and modulator elements are unknown, can be linked to a second triplet [401], in which the biological activity of both modulator and effector are known, through a common assay shared by both triplets, and consequently yields information on the possible biological activities of the modulator and effector of the first triplet [400]. By extension of the same principle triplet [402] can be linked to triplet [401] through a common modulator and further linkages to triplets [403] through [408] established. In Figure 5 such linkages are represented in a two dimensional plane, in practice linkages are not constrained to a linear branching structure and may comprise loops [L1] making further connections, branch point (B) or multiple branch points (e.g. B1, B2) from the same triplet.

### Specific Examples

### Example 1

A collection of cDNAs (Invitrogen & Image Consortium, Table 2) were prepared for expression as cDNA-EGFP fusion proteins by inserting cDNA sequences into the multiple cloning site of pCORON1000-EGFP-N2 and pCORON1000-EGFP-C1 expression vectors (Amersham Biosciences) using standard molecular cloning techniques (Molecular Cloning, Sambrook & Russell, Cold Spring Harbour Press 2001). These vectors direct the expression of fusion proteins comprising the protein encoded by the inserted cDNA sequence fused at their amino and carboxy termini to EGFP in mammalian cells under the control of a constitutively active CMV promoter.

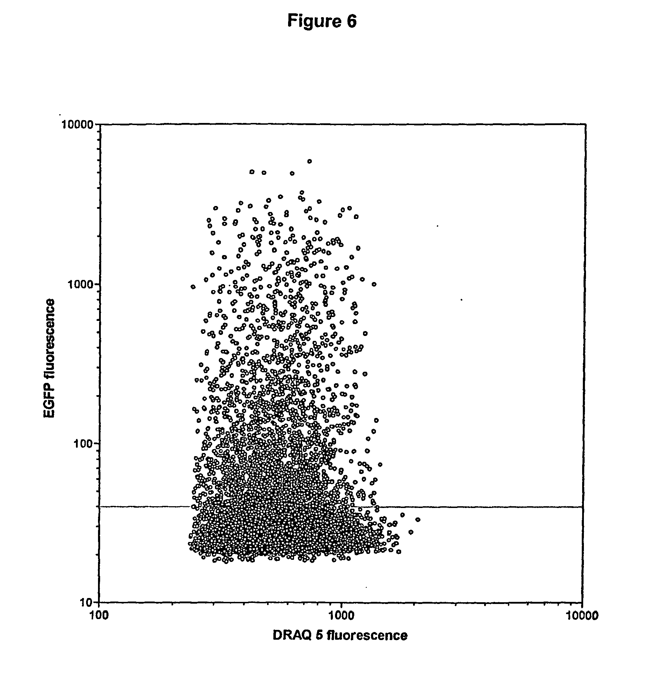

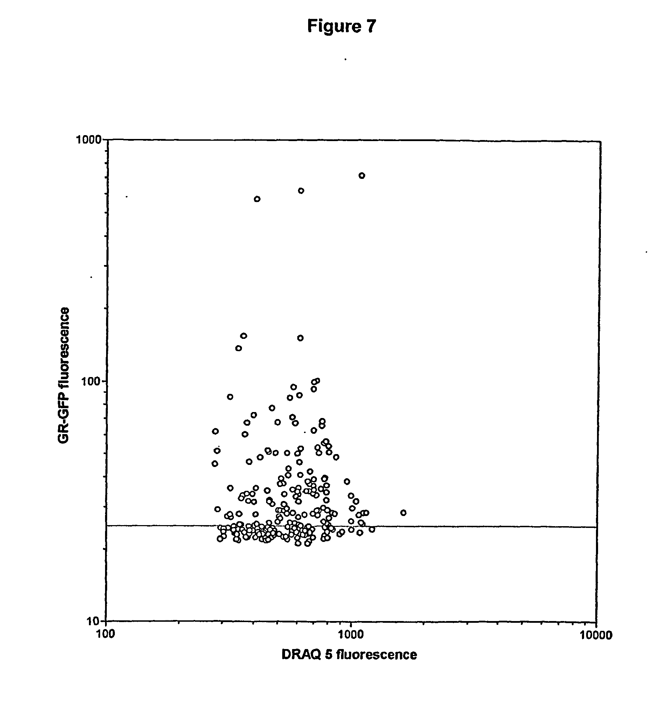

Expression vectors encoding cDNA-EGFP indicators were transiently transfected into HeLa cells growing in wells of 96 well microtitre plates by chemically mediated transfection (Fugene, Roche) and cells incubated under standard growth conditions for 24 hours to permit synthesis of indicator fusion proteins. Cells were subsequently stained with DRAQ 5, a cell permeable nuclear DNA binding dye (Biostatus), to fluorescently mark cell nuclei, and all wells imaged with dual laser excitation (EGFP 488nm, DRAQ 5 633nm) using an IN Cell Analyzer (Amersham Biosciences). Data for green (EGFP) and red (DRAQ 5) fluorescence were collected for all cells (Figure 6) and used to determine thresholds for data separation of transfected cells (EGFP fluorescence above threshold) from non-transfected cells (EGFP fluorescence below threshold). Representative data from a single cDNA-EGFP fusion protein are shown in Figure 7. A fusion protein derived from full length cDNA encoding the glucocorticoid receptor inserted in pCORON1000-EGFP-N2 was expressed in HeLa cells and analysed as described above. For this indicator protein a threshold of 25 (horizontal dotted line on Figure 7) was used to discriminate data from transfected (>25) and non-transfected cells (<25).

Data collection and analysis as described above allows cDNA-EGFP fusion proteins to be used as indicators in transiently transfected cell populations by using data thresholding to distinguish transfected from non-transfected cells, so avoiding the need to engineer stable cell lines required for analysis methods which use population average measurements.

### Example 2

Indicator proteins derived from a range of cDNAs as described for Example 1 were transfected into HeLa cells and allowed to express for 24 hours. Following expression, cells were transferred into serum-free media for 2 hours to allow effects of stimuli from serum factors such as cortisol to decay. Cells were stained with DRAQ 5, imaged as described in Example 1, returned to complete media and then exposed to 1 µM dexamethasone (a synthetic glucocorticoid agonist) or 1µM staurosporine (kinase inhibitor and apoptosis inducer) for 5 minutes followed by repeat imaging. Image data were analysed using a nuclear trafficking algorithm (Amersham Biosciences; (cf. Adie et al. (2001) The pharmacological characterisation of a GPCR using pH sensitive cyamine dyes on the LEADseeker Cell Analysis System' Poster, Society for Biomolecular Screening Conference 10-13th September 2001, Baltimore USA; Goodyer et al. (2001) 'Screening of signalling events in live cells using novel GFP redistribution assays' Poster, Society for Biomolecular Screening Conference 10-13th September 2001). The alogorithm returns a numerical description of fluorescence distribution in nucleus and cytoplasm as a ratio (nuclear fluorescence divided by cytoplasmic fluorescence ; N/C). This algorithm allows the spatial distribution of cDNA-EGFP fusion proteins to be quantitated in expressing cells: a low N/C ratio indicating a cytoplasmic location for the indicator protein, a high N/C ration indicating a nuclear location. Consequently a change in N/C ratio for an indicator protein induced by a chemical modulator indicates a translocation of the indicator in response to the modulator. This form of analysis permits screening of combinations of indicators/chemical modulators for pairings in which the indicator exhibits translocation in response to the modulator, and may serve as the basis for testing the action of effectors or further modulators on the characterised response.

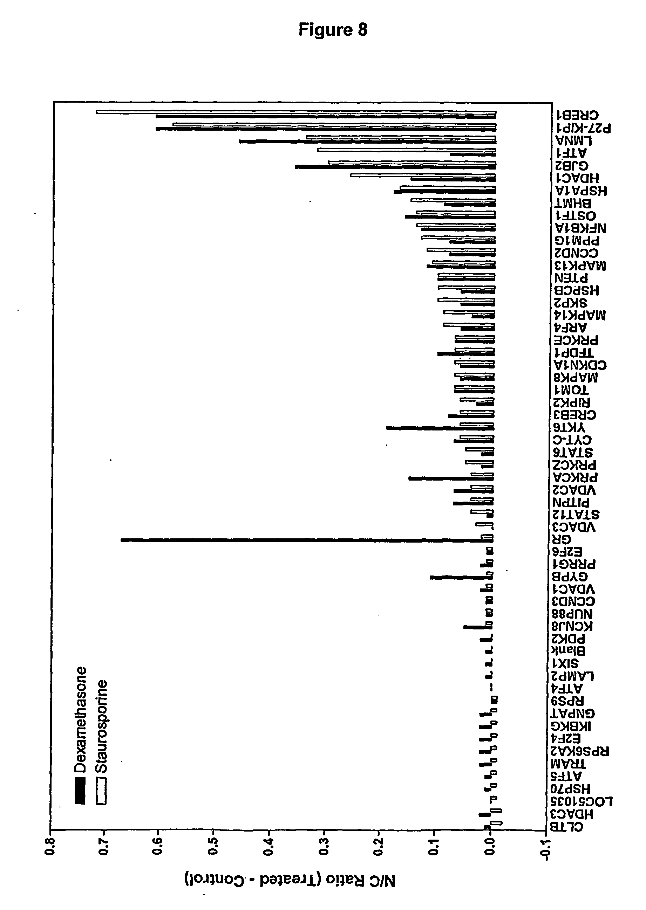

Results from this analysis are shown in Figure 8 with differences in N/C ratios in the absence and presence of dexamethasone and staurosporine plotted for a range of indicator fusion proteins. The results show a diversity of response across the indicator proteins to the two modulators used in this example. A indicator protein (GR) constructed by fusion of glucocorticoid receptor to EGFP showed a very large increase in N/C ratio indicative of a change in localisation of the indicator protein from cytoplasm to nucleus. This change in localisation is consistent with the well characterised translocation response of glucocorticoid receptor on exposure to glucocorticoid agonists, including dexamethasone (Htun et al. (1996) Proc Natl Acad Sci USA 93(10), 4845-50). A number of other indicator proteins showed a significant change in N/C ratio when exposed to either dexamethasone or staurosporine (e.g.ATF1, YKT6)

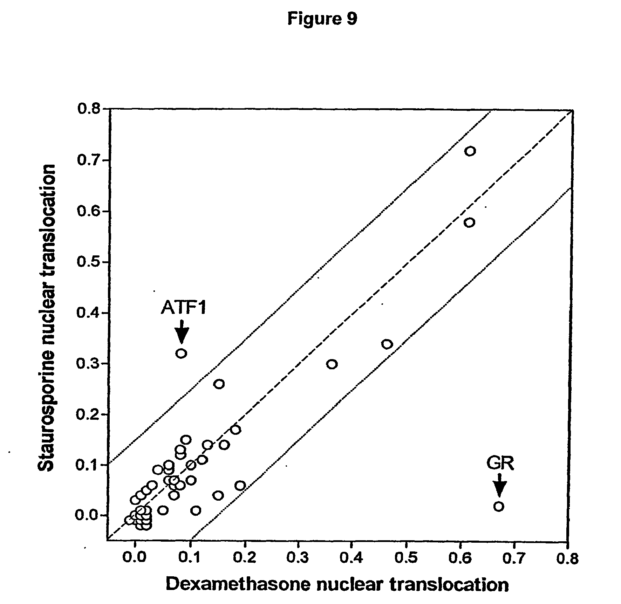

Data from this example are also shown in Figure 9 as a scatterplot of dexamethasone response against staurosporine response. Plotting data in this form highlights differential responses of indicators to modulators; most indicators either do not show a response to either modulator or show an equivalent response to both modulator treatments. When plotted in this manner the data clearly show that two indicators, GR (glucocorticoid receptor) and ATF1 (activating transcription factor 1) show specific and differential responses to the two modulators. The involvement of ATF1 in cellular response to stress has been described previously (Wiggin et al. (2002) Mol Cell Biol Apr., 22(8), 2871-81) indicating that the ATF1-staurosporine pairing would serve as a suitable test system for studying the activity of effectors or modulators on cellular stress response mechanisms. The data shown in Figure 9 also highlight those indicators which responded to both dexamethasone and staurosporine. These responses are a direct result of the serum removal and replacement regime required to measure GR translocation, where a group of indicator proteins, including CREB1, P27-KIP and LMNA show a change in N/C value following the return of cells to serum containing medium.

### Example 3

A further group of indicator proteins were transfected into HeLa cells and cells imaged before and after exposure to staurosporine as described in Example 2. Images were analysed with a further two IN Cell Analyzer algorithms, Granularity and Membrane Spot (Amersham Biosciences) (cf. Adie et al. (2001) 'The pharmacological characterisation of a GPCR using pH sensitive cyamine dyes on the LEADseeker Cell Analysis System' Poster, Society for Biomolecular Screening Conference 10-13th September 2001, Baltimore USA; Goodyer et al. (2001) 'Screening of signalling events in live cells using novel GFP redistribution assays' Poster, Society for Biomolecular Screening Conference 10-13th September 2001). These algorithms return results which quantitate fluorescence in degrees of granularity (i.e. low value indicates uniform distribution, high value indicates punctate distribution) and in terms of membrane localisation. Consequently these algorithms are suitable for examining indicators which no not exhibit cytoplasmic to nuclear differential localisation and hence are unsuitable for analysis by the algorithm used in the previous example.

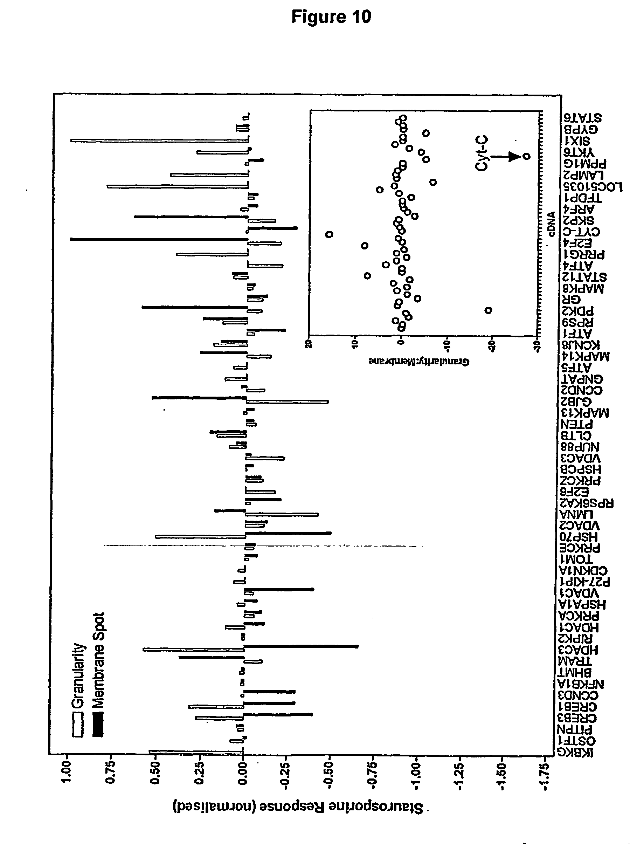

Results from analysis with these two algorithms on staurosporine treated cells are shown in Figure 10. Data returned by the algorithms varied significantly across the range of indicators, with some proteins yielding a high granularity value and a low membrane spot value, and vice versa. Examination of the ratios of the outputs from the two algorithms (Figure 10 inset) revealed that the indicator, Cyt-C (EGFP-Cytochrome C), showed the highest differential return from the two algorithms. Release of Cytochrome-C from mitochondria and subsequent cellular redistribution is a well characterised early event in the onset of cellular apoptosis (Gao et al. (2001) J Cell Sci., 114, 2855-62). Consequently, data from this example provide further evidence that indicator proteins engineered from cDNAs coding for cellular proteins fused to a detectable marker and transiently expressed in mammalian cells provide a means of gaining functional information relevant to the protein encoded by the cDNA; such indicator-modulator pairings are suitable for use in further functional screening.

### Example 4

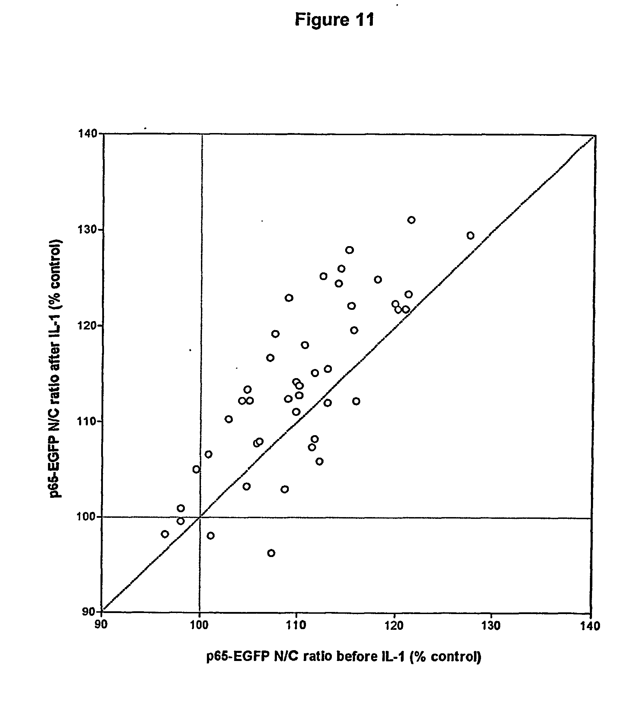

A range of cDNA modulators were transiently transfected into CHO cells expressing a NFκB p65-GFP fusion protein. This indicator undergoes a well characterised cytoplasmic to nuclear translocation in response to a number of stimuli, including exposure to Interleukin-1 (IL-1). Cells were incubated for 24 hours post transfection, stained with DRAQ 5, imaged, and then stimulated with IL-1, followed by repeat imaging. N/C ratios were determined for all images using the algorithm described in Example 2, and a scatterplot (Figure 11) prepared from the data.

In an experiment of this design where two factors (stimulus and effector) may change the behaviour of the indicator, a number of possibilities may occur;
a) the effector may decrease the indicator N/C ratio prior to stimulus relative to a control value (cells in the absence of effector)
b) the effector may increase the indicator N/C ratio prior to stimulus relative to a control value
c) the effector may decrease the indicator N/C ratio following stimulus relative to a control value
d) the effector may increase the indicator N/C ratio following stimulus relative to a control value
all of the above may, depending on their combination, result in a modulation of the magnitude of change of the indicator N/C ratio induced by IL-1 stimulus. The scatterplot of Figure 1 represents these scenarios graphically by separating results into four quadrants;

| Quadrant | Indicator behaviour |
|---|---|
| lower left | N/C₀<control: N/C_{IL-1} <control |
| lower right | N/C₀>control:N/C_{IL-1}<control |
| upper right | N/C₀>control:N/C_{IL-1}>control |
| upper left | N/C₀<control: N/C_{IL-1}>control |

In addition, the diagonal dotted line on Figure 11 indicates points of equivalent N/C ratios, consequently the distance from the line (at 90° to the line) of any value gives a measure of the overall response of the indicator protein to IL-1 stimulation in the presence of a given effector relative to the absence of the effector. It is clear that the effectors used in this experiment are having a range of effects on the distribution of the indicator protein in changing the N/C ratio before and after IL-1 stimulus and in changing the overall response to IL-1 stimulation.

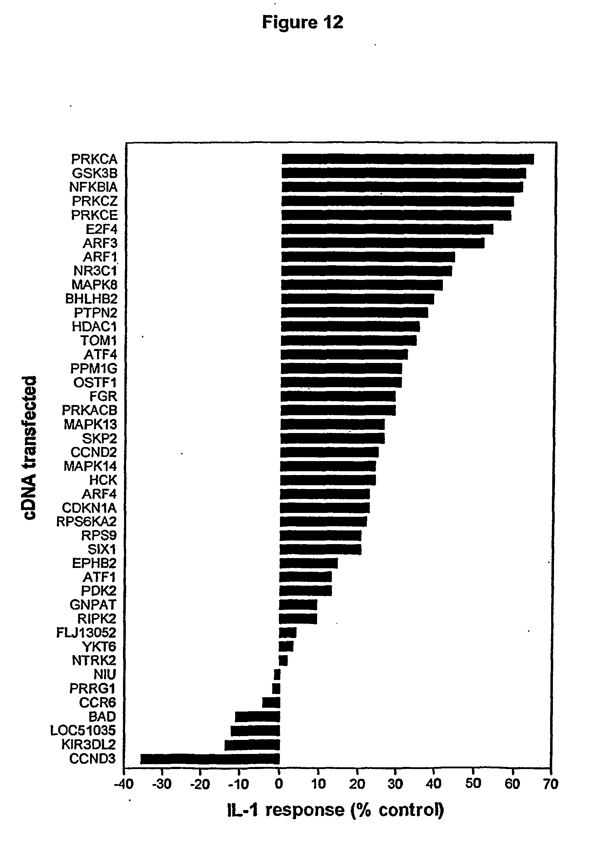

Figure 12 shows a simplified treatment of these results where only data for IL-1 response (i.e. the difference between N/C₀ and N/C_{IL-1}) are shown. These data indicate a range of responses to transfection with effectors ranging from significant antagonism of IL-1 stimulation (CCND3) to strong agonism (e.g. PRKCs A, Z & E and GSK3B). These agonists have previously been shown to modulate the activity of the NFκB signalling pathway (La Porta et al. (1998) Anticancer Res. 18(4A):2591-7; Hoeflich et al. (2000) Nature 406 (6791), 86-90) confirming the validity of using this approach for functional screening of cDNA effectors against indicators expressed in mammalian cells.

### Example 5

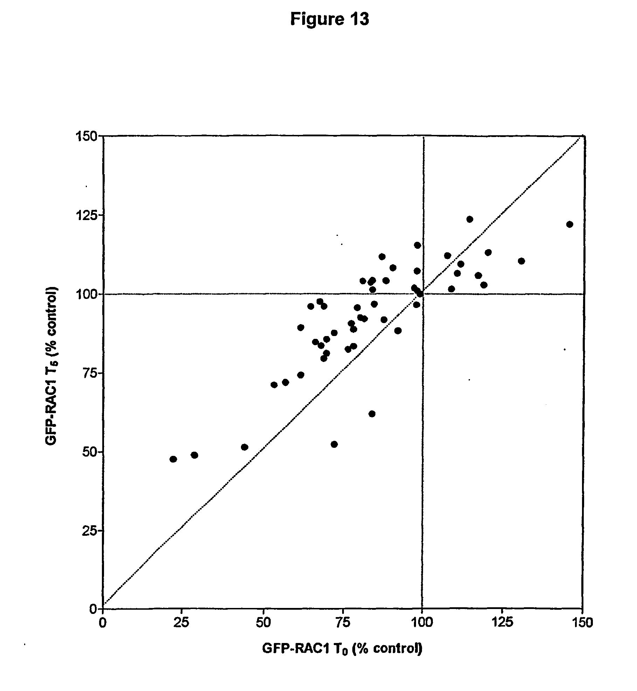

The functional screen of Example 4 was repeated with a second indicator, RAC1 (T)-GFP, in the presence and absence of stimulation with insulin and analysed using the membrane spot algorithm described in Example 3. As in Example 4 it is clear that the effectors used in this experiment are having a range of effects on the distribution of the indicator protein in changing the cellular distribution of the indicator both before and after insulin stimulus and in changing the overall response to insulin stimulation (Figure 13).

**Table 1**

| | Identity or Function | | |
|---|---|---|---|
| | **modulator** | **effector** | **indicator** |
| [302] | known | known | unknown |
| [303] | known | unknown | known |
| [304] | unknown | known | known |
| [305] | known | unknown | unknown |
| [306] | unknown, | unknown | known |
| [307] | unknown | known | unknown |
| [308] | known | known | known |
| [309] | unknown | unknown | unknown |

**Table 2**

| | | | | | |
|---|---|---|---|---|---|
| cDNA | Genbank No. | cDNA | Genbank No. | cDNA | Genbank No. |
| IKBKG | NM_003639 | PDK2 | L42451 | AGPAT2 | AF000237 |
| NFKBIA | M69043 | VDAC1 | BC008482 | ICAM2 | X15606 |
| PRKCA | X52479 | VDAC2 | BC012883 | CCR6 | U45984 |
| PRKCE | X65293 | VDAC3 | BC002456 | NTRK2 | X72958 |
| PRKCZ | L14283 | CCND2 | M90813 | HCK | M16591 |
| MAPK13 | AF004709 | CCND3 | M92287 | EPHB2 | L41939 |
| MAPK14 (p38) | L35253 | RPS6KA2 | X85106 | KIR3DL2 | L41270 |
| MAPK8 | L26318 | ATF1 | X55544 | AP1M2 | BC003612 |
| PRKACB | M34181 | ATF4 | D90209 | APBB1 | BC010854 |
| SKP2 (p45) | U33761 | CDKN1A | L25610 | APP | BC004369 |
| PPM1G | Y13936 | HDAC1 | D50405 | AQP3 | BC013566 |
| FGR (src) | M19722 | TFDP1 | L23959 | CLTA | BC009201 |
| GSK3B | L33801 | E2F4 | S75174 | CLTB | BC006457 |
| OSTF1 | U63717 | SIX1 | X91868 | GABRA5 | BC011403 |
| BHMT | U50929 | ATF5 | BC005174 | GABRB3 | BC010641 |
| HSPA1A (hsp70) | M11717 | CREB1 | BC010636 | GJB2 | BC002805 |
| PTPN2 | M25393 | CREB3 | BC010158 | KCNH2 | BC001914 |
| BHLHB2 | AB004066 | DUSP4 | BC002671 | KCNJ8 | BC000544 |
| BAD | U66879 | E2F6 | BC008348 | KCNQ2 | BC000699 |
| MYBPH | U27266 | HDAC3 | BC000614 | P2RX7 | BC011913 |
| ACTB | BC002409 | HIF1A | BC012527 | STATI2 | BC010399 |
| AKT1 | BC000479 | P27-KIP1 | BC001971 | OPRD1 | NM_000911 |
| ARAF1 | BC007514 | LMNA | BC000511 | PTGIR | NM_000960 |
| ARRB1 | BC003636 | NUP88 | BC000335 | AGTR2 | NM_000686 |
| ARRB2 | BC007427 | LAMP2 | BC002965 | CHRM3 | NM_000740 |
| BID | BC009197 | GNPAT | AJ002190 | CHRM1 | NM_000738 |
| FADD | BC000334 | RPS9 | U14971 | EGFR | NM_005228 |
| HSP70 | BC002453 | PRRG1 | AF009242 | ARF 1 | M36340 |
| HSPCB(hsp90) | BC009206 | LOC51035 | M68864 | ARF 3 | M74491 |
| MADH1 | BC001878 | NOT IN UNIGENE | D14825 | ARF 4 | M36341 |
| MADH4 | BC002379 | FLJ13052 | M37712 | YKT6 | U95735 |
| MAPK7 | BC007992 | PLCG2 | BC007565 | PITPN | D30036 |
| MDM2 | BC009893 | RIPK2 | AF027706 | TOM1 | NM_005488 |
| MYCBP | BC008686 | GYPB | J02982 | TRAM | BC000687 |
| NFATC3 | BC001050 | PROC | X02750 | STAT6 | BC004973 |
| PSCD2 STAT3 | BC004361 BC000627 | PTEN | BC005821 | TRADD | BC004491 |

## Claims

1. A method for determining the function or effect of a genetic element or a chemical modulator from libraries of said genetic elements and chemical modulators of known and unknown function on a population of cells comprising
i) determining the distribution of a detectable label expressed from an indicator nucleic acid sequence in said cells in the presence and the absence of a first chemical modulator, which modulator affects said distribution of said
indicator, wherein the cells are both co-expressing a library of effector nucleic acid sequences and are in the presence of a library of second chemical modulators; and
ii) analysing the distribution data from all combinations of said effector, modulator and indicator to derive functional linkages and assign function to the effector and said second modulator.

2. A method for determining the function or effect of a genetic element or a chemical modulator from libraries of said genetic elements and chemical modulators of known and unknown function on a population of cells comprising
i) determining the distribution of a detectable label expressed from an indicator nucleic acid sequence in said cells in the presence of a first chemical modulator, which modulator affects said distribution of said indicator, wherein the cells are both co-expressing a library of effector nucleic acid sequences and are in the presence of a library of second chemical modulators;
ii) comparing the distribution data of i) above with known distribution data, stored on an electronic or optical database, for the detectable label expressed from said indicator nucleic acid sequence in the absence of said first chemical modulator; and
iii) analysing the distribution data from all combinations of said effector, modulator and indicator to derive functional linkages and assign function to the effector and said second modulator.

3. The method according to either of claims 1 or 2, wherein the effector nucleic acid sequence encodes a protein or peptide and is selected from the group consisting of DNA, cDNA, RNA and Protein Nucleic Acid.

4. The method according to any of claims 1 to 3, wherein the effector nucleic acid is an antisense oligonucleotide.

5. The method according to any of claims 1 to 3, wherein the effector nucleic acid is a small interfering RNA (siRNA) which causes gene silencing.

6. The method according to any of claims 1 or 5, wherein the effector nucleic acid comprises a nucleic acid sequence in a cellular expression vector.

7. The method of claim 6, wherein said expression vector is selected from the group consisting of plasmid, retrovirus and adenovirus.

8. The method according to claim 7, wherein the indicator nucleic acid sequence is created by fusing said nucleic acid sequence to a nucleic acid sequence encoding a detectable label.

9. The method according to any of claims 1 to 8, wherein said detectable label is selected from the group consisting of fluorescent protein, enzyme, antigen and antibody.

10. The method according to claim 9, wherein said fluorescent protein is a modified Green Fluorescent Protein (GFP) having one or more mutations selected from the group consisting of Y66H, Y66W, Y66F, S65T, S65A, V68L, Q69K, Q69M, S72A, T2031, E222G, V163A, I167T, S175G, F99S, M153T, V163A, F64L, Y145F, N149K, T203Y, T203Y, T203H, S202F and L236R.

11. The method according to claim 10, wherein said modified GFP has three mutations selected from the group consisting of F64L-V163A-E222G, F64L-S175G-E222G, F64L-S65T-S175G and F64L-S65T-V163.

12. The method according to claim 9, wherein said enzyme is selected from the group consisting of β-galactosidase, nitroreductase, alkaline phosphatase and β-lactamase.

13. The method according to any of claims 1 to 12, wherein the modulator is selected from the group consisting of organic compound, inorganic compound, peptide, polypeptide, protein, carbohydrate, lipid, nucleic acid, polynucleotide and protein nucleic acid.

14. The method according to any of claims 1 to 13, wherein the modulator is selected from a combinatorial library comprising similar organic compounds such as analogues or derivatives.

15. The method according to any of claims 1 to 14, wherein said cell is an eukaryotic cell.

16. The method according to claim 15, wherein said eukaryotic cell is selected from the group consisting of mammal, plant, bird, fungus, fish and nematode, which cell may or may not be genetically modified.

17. The method according to claim 16, wherein said mammalian cell is a human cell, which cell may or may not be genetically modified.

18. The method according to any of claims 1 to17, wherein the distribution of the detectable label is determined using an imaging system.

## Patentansprüche

1. Verfahren zur Bestimmung der Funktion oder Wirkung eines genetischen Elements oder eines chemischen Modulators aus Bibliotheken der genetischen Elemente und chemischen Modulatoren bekannter und unbekannter Funktion auf einer Zellpopulation, umfassend
i) Bestimmen der Verteilung einer detektierbaren Markierung, die von einer Indikator-Nukleinsäuresequenz in den Zellen in Anwesenheit und Abwesenheit eines ersten chemischen Modulators exprimiert wird, welcher Modulator die Verteilung des Indikators beeinflusst, wobei die Zellen beide eine Bibliothek von Effektor-Nukleinsäuresequenzen koexprimieren und in Gegenwart einer Bibliothek zweiter chemischer Modulatoren sind; und
ii) Analysieren der Verteilungsdaten aus allen Kombinationen des Effektors, Modulators und Indikators, um funktionelle Kopplungen abzuleiten und dem Effektor und dem zweiten Modulator eine Funktion zuzuordnen.

2. Verfahren zur Bestimmung der Funktion oder Wirkung eines genetischen Elements oder eines chemischen Modulators aus Bibliotheken der genetischen Elemente und chemischen Modulatoren bekannter und unbekannter Funktion auf einer Zellpopulation, umfassend
i) Bestimmen der Verteilung einer detektierbaren Markierung, die von einer Indikator-Nukleinsäuresequenz in den Zellen in Anwesenheit eines ersten chemischen Modulators exprimiert wird, welcher Modulator die Verteilung des Indikators beeinflusst, wobei die Zellen beide eine Bibliothek von Effektor-Nukleinsäuresequenzen koexprimieren und in Gegenwart einer Bibliothek zweiter chemischer Modulatoren sind;
ii) Vergleichen der Verteilungsdaten von i) oben mit bekannten Verteilungsdaten, die in einer elektronischen oder optischen Datenbank gespeichert sind, bezüglich der detektierbaren Markierung, die von der Indikator-Nukleinsäuresequenz in Abwesenheit des ersten chemischen Modulators exprimiert wird; und
iii) Analysieren der Verteilungsdaten aus allen Kombinationen des Effektors, Modulators und Indikators, um funktionelle Kopplungen abzuleiten und dem Effektor und dem zweiten Modulator eine Funktion zuzuordnen.

3. Verfahren nach Anspruch 1 oder 2, wobei die Effektor-Nukleinsäuresequenz für ein Protein oder Peptid codiert und aus jener Gruppe ausgewählt ist, die sich aus DNA, cDNA, RNA und Proteinnukleinsäure zusammensetzt.

4. Verfahren nach einem der Ansprüche 1 bis 3, wobei die Effektor-Nukleinsäure ein Antisense-Oligonukleotid ist.

5. Verfahren nach einem der Ansprüche 1 bis 3, wobei die Effektor-Nukleinsäure eine kurze interferierende RNA (siRNA) ist, welche eine Gen-Stilllegung bewirkt.

6. Verfahren nach einem der Ansprüche 1 bis 5, wobei die Effektor-Nukleinsäure eine Nukleinsäuresequenz in einem zellulären Expressionsvektor umfasst.

7. Verfahren nach Anspruch 6, wobei der Expressionsvektor aus der Gruppe ausgewählt ist, die sich aus Plasmid, Retrovirus und Adenovirus zusammensetzt.

8. Verfahren nach Anspruch 7, wobei die Indikator-Nukleinsäuresequenz erzeugt wird durch Fusionieren der Nukleinsäuresequenz an eine Nukleinsäuresequenz, die für eine detektierbare Markierung codiert.

9. Verfahren nach einem der Ansprüche 1 bis 8, wobei die detektierbare Markierung aus der Gruppe ausgewählt ist, die sich aus fluoreszierendem Protein, Enzym, Antigen und Antikörper zusammensetzt.

10. Verfahren nach Anspruch 9, wobei das fluoreszierende Protein ein modifiziertes grün fluoreszierendes Protein (GFP) mit einer oder mehreren Mutationen ist, ausgewählt aus der Gruppe, die sich zusammensetzt aus Y66H, Y66W, Y66F, S65T, S65A, V68L, Q69K, Q69M, S72A, T203I, E222G, V163A, I167T, S175G, F99S, M153T, V163A, F64L, Y145F, N149K, T203Y, T203Y, T203H, S202F und L236R.

11. Verfahren nach Anspruch 10, wobei das modifizierte GFP drei Mutationen aufweist, die aus der Gruppe ausgewählt sind, die sich aus F64L-V163A-E222G, F64L-S175G-E222G, F64L-S65T-S175G und F64L-S65T-V163 zusammensetzt.

12. Verfahren nach Anspruch 9, wobei das Enzym aus der Gruppe ausgewählt ist, die sich aus β-Galaktosidase, Nitroreduktase, alkalischer Phosphatase und β-Laktamase zusammensetzt.

13. Verfahren nach einem der Ansprüche 1 bis 12, wobei der Modulator aus der Gruppe ausgewählt ist, die sich zusammensetzt aus organischer Verbindung, anorganischer Verbindung, Peptid, Polypeptid, Protein, Kohlenhydrat, Lipid, Nukleinsäure, Polynukleotid und Proteinnukleinsäure.

14. Verfahren nach einem der Ansprüche 1 bis 13, wobei der Modulator aus einer kombinatorischen Bibliothek ausgewählt ist, welche ähnliche organische Verbindungen, wie z.B. Analoge oder Derivate, umfasst.

15. Verfahren nach einem der Ansprüche 1 bis 14, wobei die Zelle eine eukaryotische Zelle ist.

16. Verfahren nach Anspruch 15, wobei die eukaryotische Zelle aus der Gruppe ausgewählt ist, die sich zusammensetzt aus Säuger, Pflanze, Vogel, Pilz, Fisch und Nematode, welche Zelle genetisch modifiziert sein kann oder nicht.

17. Verfahren nach Anspruch 16, wobei die Säugerzelle eine menschliche Zelle ist, welche Zelle genetisch modifiziert sein kann oder nicht.

18. Verfahren nach einem der Ansprüche 1 bis 17, wobei die Verteilung der detektierbaren Markierung mittels eines Bildgebungssystems bestimmt wird.

## Revendications

1. Procédé de détermination de la fonction ou de l'effet d'un élément génétique ou d'un modulateur chimique à partir de librairies desdits éléments génétiques et de modulateurs chimiques de fonctions connue et inconnue sur une population de cellules comprenant:
i) la détermination de la répartition d'une étiquette détectable exprimée à partir d'une séquence d'acides nucléiques d'indicateur dans lesdites cellules en la présence et en l'absence d'un premier modulateur chimique, lequel modulateur affecte ladite répartition dudit indicateur, dans lequel les cellules expriment simultanément à la fois une librairie de séquences d'acides nucléiques d'effecteur et sont en la présence d'une librairie de seconds modulateurs chimiques ; et
ii) l'analyse des données de répartition à partir de toutes les combinaisons desdits effecteur, modulateur et indicateur afin de déduire des liaisons fonctionnelles et d'assigner une fonction à l'effecteur et audit second modulateur.

2. Procédé de détermination de la fonction ou de l'effet d'un élément génétique ou d'un modulateur chimique à partir de librairies desdits éléments génétiques et de modulateurs chimiques de fonction connue et inconnue sur une population de cellules comprenant:
i) la détermination de la répartition d'une étiquette détectable exprimée à partir d'une séquence d'acides nucléiques d'indicateur dans lesdites cellules en la présence d'un premier modulateur chimique, lequel modulateur affecte ladite répartition dudit indicateur, dans lequel les cellules expriment simultanément à la fois une librairie de séquences d'acides nucléiques d'effecteur et sont en la présence d'une librairie de seconds modulateurs chimiques ;
ii) la comparaison des données de répartition de i) ci-dessus à des données de répartition connues, mémorisées sur une base de données électronique ou optique, de l'étiquette détectable exprimée à partir de ladite séquence d'acides nucléiques d'indicateur en l'absence dudit premier modulateur chimique ;et
iii) l'analyse des données de répartition à partir de toutes les combinaisons desdits effecteur, modulateur et indicateur afin de déduire des liaisons fonctionnelles et d'assigner une fonction à l'effecteur et audit second modulateur.

3. Procédé selon l'une ou l'autre des revendications 1 ou 2, dans lequel la séquence d'acides nucléiques d'effecteur code une protéine ou un peptide et est sélectionnée dans le groupe constitué par l'ADN, l'ADN c, l'ARN et l'acide nucléique de protéine.

4. Procédé selon l'une quelconque des revendications 1 à 3, dans lequel l'acide nucléique d'effecteur est un oligonucléotide antisens.

5. Procédé selon l'une quelconque des revendications 1 à 3, dans lequel l'acide nucléique d'effecteur est un petit ARN interférant (ARNpi) qui provoque le silence de gène.

6. Procédé selon l'une quelconque des revendications 1 ou 5, dans lequel l'acide nucléique d'effecteur comprend une séquence d'acide nucléique dans un vecteur d'expression cellulaire.

7. Procédé selon la revendication 6, dans lequel ledit vecteur d'expression est sélectionné dans le groupe constitué par un plasmide, un rétrovirus et un adénovirus.

8. Procédé selon la revendication 7, dans lequel la séquence d'acide nucléique d'indicateur est créée en fusionnant ladite séquence d'acide nucléique à une séquence d'acide nucléique codant une étiquette détectable.

9. Procédé selon l'une quelconque des revendications 1 à 8, dans lequel ladite étiquette détectable est sélectionnée dans le groupe constitué par une protéine fluorescente, une enzyme, un antigène et un anticorps.

10. Procédé selon la revendication 9, dans lequel ladite protéine fluorescente est une protéine fluorescente verte (GFP) modifiée comportant une ou plusieurs mutations sélectionnées dans le groupe constitué par Y66H, Y66W, Y66F, S65T, S65A, V68L, Q69K, Q69M, S72A, T203I, E222G, V163A, I167T, S175G, F99S, M153T, V163A, F64L, Y145F, N149K, T203Y, T203Y, T203H, S202F et L236R.

11. Procédé selon la revendication 10, dans lequel ladite protéine GFP modifiée comporte trois mutations sélectionnées dans le groupe constitué par F64L-V163A-E222G, F64L-S175G-E222G, F64L-S65T-S175G et F64L-S65T-V163.

12. Procédé selon la revendication 9, dans lequel ladite enzyme est sélectionnée dans le groupe constitué par la β-galactosidase, la nitroréductase, la phosphatase alcaline et la β-lactamase.

13. Procédé selon l'une quelconque des revendications 1 à 12, dans lequel le modulateur est sélectionné dans le groupe constitué par un composé organique, un composé inorganique, un peptide, un polypeptide, une protéine, un hydrate de carbone, un lipide, un acide nucléique, un polynucléotide et un acide nucléique de protéine.

14. Procédé selon l'une quelconque des revendications 1 à 13, dans lequel le modulateur est sélectionné à partir d'une librairie combinatoire comprenant des composés organiques similaires tels que des analogues ou des dérivés.

15. Procédé selon l'une quelconque des revendications 1 à 14, dans lequel ladite cellule est une cellule eucaryote.

16. Procédé selon la revendication 15, dans lequel ladite cellule eucaryote est sélectionnée dans le groupe constitué par un mammifère, une plante, un oiseau, un champignon, un poisson et un nématode, laquelle cellule pouvant être génétiquement modifiée ou non.

17. Procédé selon la revendication 16, dans lequel ladite cellule de mammifère est une cellule humaine, laquelle cellule pouvant être génétiquement modifiée ou non.

18. Procédé selon l'une quelconque des revendications 1 à 17, dans lequel la répartition de l'étiquette détectable est déterminée en utilisant un système d'imagerie.
